# FASCICULE DE BREVET EUROPEEN

(11) **EP 0 810 222 B1**
(45) Date de publication et mention de la délivrance du brevet: **13.06.2001**
(21) Numéro de dépôt: 97400589.4
(22) Date de dépôt: 17.03.1997
(51) Int. Cl.: C07D 311/62

(54) **Procédé d'extraction de polyphénols catéchiques à partir de potentilles, extrait obtenu et son utilisation**
Verfahren zur Extrahierung von polyphenolischen Verbindungen von Catechin-Typ aus pflanzenartigen Quellen, die erhaltene Extrakte und deren Verwendung
Process for extraction of polyphenolic compounds of catechin type from plants, the so obtained extract and its use

(30) Priorité: 30.05.1996 FR 9606649
(43) Date de publication de la demande: 03.12.1997
(73) Titulaire: BERKEM, 24680 Gardonne (FR)
(72) Inventeur: Nkiliza, Jean, 33220 Port Sainte Foy (FR)
(74) Mandataire: Peuscet, Jacques

(56) Documents cités:
- EP-A- 0 707 005
- GB-A- 884 184

## Description

La présente invention concerne un procédé d'extraction de polyphénols catéchiques à partir de différentes espèces de potentille, l'extrait susceptible d'être obtenu par ce procédé et l'utilisation de l'extrait obtenu comme composé actif dans la préparation de compositions pharmaceutiques, cosmétiques et diététiques.

Il est connu que de nombreuses plantes contiennent des polyphénols à hétérocycles oxygénés, dits "flavonoïdes" au sens large, tels que des catéchines (proanthocyanidols), des flavanones, des flavonols, des anthocyanes et similaires. La présente invention concerne un procédé d'extraction, à partir de potentille, de polyphénols catéchiques ou dérivés de flavanol-3, parmi lesquels on peut citer l'épicatéchol, le catéchol et des oligomères de ces composés, en particulier les procyanidols.

Selon l'invention, l'extraction de ces polyphénols catéchiques est effectuée à partir de différentes espèces de potentille. Parmi ces espèces, on peut citer la tormentille (Potentilla tormentilla Neck, également appelée Tormentilla erecta L., Tormentilla reptans L., Tormentilla officinalis Curt, Potentilla tetrapetala Hall, Fragaria tormentilla Granz), l'ansérine (Potentilla anserina L. ou Argentina Vulgaris Lam.) et la quintefeuille ou potentille rampante (Potentilla reptans L.).

Les différentes espèces de potentille et, en particulier, la tormentille ont déjà été utilisées en médecine traditionnelle. Elles étaient administrées sous des formes diverses : en poudre, décoction ou macération ; elles étaient utilisées pour traiter les affections inflammatoires aigües ou chroniques des muqueuses gastro-intestinales, ou comme antiseptiques sous forme de bains de bouche ou gargarismes, ou encore pour soigner les pharyngites.

Par ailleurs, des travaux scientifiques ont mis en évidence la présence de polyphénols catéchiques dans différentes espèces de potentille, en particulier Potentilla erecta L. Sur ce point, on peut citer C.A. 63, 9742 h, qui indique la présence de tanins dans Potentilla erecta ; C.A. T 63, 10314 e, qui indique la présence de myricétine et de leucodelphinidine dans Potentilla erecta et la présence d'acide ellagique dans pratiquement toutes les espèces de potentille ; C.A. 71, 10266 w, qui indique la présence de (+) catéchol dans Potentilla fruticosa et Potentilla anserina et la présence de (-) épicatéchol dans Potentilla anserina.

Les propriétés des potentilles utilisées en médecine traditionnelle sont probablement dues à la présence des polyphénols catéchiques. Par ailleurs, il est connu d'utiliser les polyphénols catéchiques, par exemple extraits d'écorce de pin, en thérapeutique comme veinotonique, comme cofacteur de vitamine C, ou pour améliorer la circulation sanguine. Ces polyphénols ont, de façon connue, des propriétés antiradicalaires, ce qui permet de les utiliser en diététique comme complément alimentaire pour prévenir l'athérosclérose et en cosmétologie pour lutter contre le vieillissement cellulaire ou l'apparition de cancers cutanés. De plus, les polyphénols catéchiques peuvent servir comme photoprotecteurs grâce à leur bonne absorption de la lumière ultraviolette.

Plusieurs procédés d'extraction de polyphénols catéchiques à partir de divers végétaux, tels que les pépins de raisins et l'écorce de pin, ont été décrits dans US-A-4 698 360, EP-A-348 781, EP-A-283 349, FR-A-1 427 100, FR-A-2 092 743, FR-A-2 643 073 et FR-A-2 372 823. Selon ces documents, on traite le végétal par l'eau (en particulier l'eau chaude) ou un mélange d'eau et d'une cétone dans des proportions variables, puis on traite l'extrait obtenu à l'aide d'une solution concentrée de sel pour précipiter les tanins à poids moléculaire élevés dépourvus d'intérêt thérapeutique ; mais l'utilisation d'une solution saline nuit à l'environnement et constitue un surcoût pour le produit fini. Dans la demande de brevet EP-A-0 707 005, il est décrit un procédé d'obtention d'oligomères procyanidoliques par traitement de pépins de raisins au moyen d'un mélange extracteur comprenant de l'eau, un solvant organique miscible à l'eau et capable de dissoudre lesdits oligomères, tel que de l'acétone, et du chlorure de sodium. On a aussi proposé d'effectuer la purification par ultrafiltration sur des membranes appropriées. Tous ces procédés ne permettent cependant pas d'obtenir à partir de potentilles un extrait riche en polyphénols catéchiques, en particulier riche en oligomères catéchiques, qui présente un degré de pureté suffisamment élevé pour pouvoir être utilisé chez l'homme.

La présente invention a pour but de proposer un procédé d'extraction de polyphénols catéchiques à partir de potentilles, qui ne comporte pas d'étape d'extraction à l'eau, pas d'étape de précipitation des tanins condensés à l'aide d'une solution de chlorure de sodium ni d'étape de purification par ultrafiltration, et qui permet d'obtenir un extrait riche en composés polyphénoliques catéchiques de pureté suffisamment élevée pour être utilisé chez l'homme.

La présente invention a donc pour objet un procédé d'extraction de polyphénols catéchiques essentiellement oligomères à partir de potentilles, caractérisé par le fait qu'il comporte les étapes suivantes :
a) la plante entière ou une partie de la plante est traitée par un solvant organique polaire, pur ou en mélange avec de l'eau,
b) l'extrait organique obtenu à l'étape a) est évaporé à sec à une température d'au plus 60°C,
c) le résidu d'évaporation de l'étape b) est repris par l'eau, puis la solution aqueuse obtenue est épuisée par un solvant non-miscible à l'eau et susceptible de dissoudre les polyphénols catéchiques oligomères,
d) la solution organique de l'étape c) est évaporée à sec à une température d'au plus 60°C, de façon à éliminer le solvant et obtenir un extrait sec.

Selon la présente invention, à l'étape a), le solvant polaire utilisé est, de préférence, un alcool aliphatique en C₁-C₄, en particulier le méthanol, l'éthanol, le propanol ou le butanol. Lorsque le solvant est mélangé avec de l'eau, il contient, de préférence, au plus 50 % en volume d'eau ; les proportions d'eau sont généralement comprises entre 1 et 30 % en volume d'eau.

Le traitement de l'étape a) est, de préférence, effectué à une température ne dépassant pas 50°C à pression atmosphérique, de façon à éviter la détérioration de l'extrait d'origine végétale.

Le traitement peut être effectué sans agitation, mais il est effectué, de préférence, sous agitation. La durée de contact entre la substance végétale et le solvant doit être suffisante pour épuiser la substance végétale. Elle est, en général, comprise entre 10 mn et 2 heures ; un temps de contact d'environ 1 heure est la plupart du temps approprié.

On peut effectuer le traitement sur la plante entière ou sur une partie de la potentille, en particulier le rhizome. On utilise, de préférence, dans le cas de l'ansérine, la plante entière, et, dans le cas de la tormentille et de la quintefeuille, le rhizome. Le choix de la partie de la plante traitée est fonction de sa teneur en polyphénols.

Avant traitement par le solvant. la potentille ou partie de potentille traitée est, de préférence, broyée.

Dans l'étape b) et/ou l'étape d). l'évaporation est effectuée, de préférence, sous pression réduite.

Dans l'étape c), le solvant non-miscible à l'eau et susceptible de dissoudre les polyphénols catéchiques oligomères est, de préférence, l'acétate d'éthyle, mais pourrait également être l'acétate de méthyle ou l'acétate de propyle. Le solvant, en particulier l'acétate d'éthyle, est, de préférence, utilisé en un volume sensiblement équivalent à celui de la phase aqueuse et plusieurs extractions successives sont effectuées. En général, quatre extractions successives sont suffisantes pour épuiser la phase aqueuse. Les phases solvant sont ensuite réunies et séchées, de préférence sur un desséchant inerte, tel que le sulfate de sodium ou de magnésium anhydre. De préférence, après reprise par l'eau du résidu d'évaporation de l'étape b), la solution aqueuse obtenue est filtrée avant d'être épuisée par le solvant non-miscible à l'eau.

A l'étape d), l'extrait obtenu après évaporation à sec est, de préférence, purifié par reprise par de l'eau distillée et ensuite séché. Le séchage se fait avantageusement par lyophilisation ou par atomisation.

L'extrait sec obtenu au stade d) est une poudre qui a une couleur beige et qui est entièrement soluble dans l'eau.

La présente invention a également pour objet l'extrait susceptible d'être obtenu par le procédé ci-dessus défini.

Cet extrait peut être utilisé comme composé actif dans la préparation de compositions pharmaceutiques, diététiques ou cosmétiques ayant une action antiradicaux libres et ou une action de protection contre les rayons ultra-violets (UV). Leur richesse en polyphénols catéchiques permet, en effet, de les utiliser pour la préparation de compositions, dont l'effet repose sur leur effet capteur envers les radicaux libres oxygénés, en particulier à usage dermatologique.

On peut, notamment, utiliser l'extrait de potentille selon l'invention en mélange avec au moins un acide hydroxycinnamique d'origine végétale décrit dans FR-A-2 734 478, pour obtenir une protection contre les agressions UV par l'effet combiné de propriétés antiradicalaires et de propriétés filtrantes car il a un très large spectre d'absorption des rayons UV couvrant la zone comprise entre 270 et 360 nm. Le mélange peut être utilisé avant, pendant ou après l'exposition aux rayons UV et être administré par voie orale ou topique. L'extrait de potentille obtenu par le procédé selon l'invention peut être utilisé en mélange avec le(s) acide(s) hydroxycinnamique(s) dans des proportions allant de 1 % à 80 % en poids d'acide(s) par rapport au poids total de la composition. Le(s) acide(s) additionnel(s) peu(ven)t être l'acide chlorogénique et ses isomères, l'acide sinapique, l'acide férulique, l'acide p-coumarique et l'acide caféique. Le(s) acide(s) hydrocycinnamique(s) additionnel(s) est (sont), de préférence, extrait(s) du café vert.

L'exemple donné ci-après, à titre purement indicatif et non limitatif permettra de mieux comprendre l'invention.

### EXEMPLE :

### A - Préparation de l'extrait

Des rhizomes de tormentille broyés à sec (17 kg) sont traités sous agitation à l'aide de 170 litres de méthanol pur, le mélange étant maintenu à 50°C. Après une durée de contact d'environ 1 heure, le mélange est filtré et le filtrat méthanolique est évaporé à sec sous une pression de 2 000 Pa par chauffage à une température d'environ 40°C. Le résidu est ensuite repris par 16 litres d'eau et filtré. La solution aqueuse obtenue est épuisée par l'acétate d'éthyle.

On a effectué quatre extractions successives en utilisant à chaque extraction un volume d'acétate d'éthyle équivalent à celui de la phase aqueuse. Les phases acétate d'éthyle sont réunies puis séchées sur 2 kg de sulfate de sodium anhydre. L'acétate d'éthyle est ensuite éliminé par évaporation sous une pression de 2 000 Pa à une température d'environ 40°C.

Le résidu obtenu est dissous dans 6 litres d'eau distillée et la solution aqueuse obtenue est séchée par lyophilisation. On obtient 0,6 kg d'une poudre beige entièrement soluble dans l'eau.

### B - Analyse des constituants de l'extrait obtenu en A

Une fraction de l'extrait obtenu en A est peracétylée par l'action de la pyridine et de l'anhydride acétique, à l'abri de la lumière et à température ambiante pendant 24 heures.

On effectue une chromatographie sur couche mince de silice du produit peracétylé, en utilisant, comme éluant, le mélange toluène/acétone dans un rapport 8:2 en volume et, comme révélateur, la lampe UV à 254 nm. On a observé trois taches principales.

On a effectué ensuite une séparation d'une fraction de l'extrait sur colonne de silice avec élution par le chloroforme et on a isolé les produits correspondant aux trois taches. On a déterminé leur structure par résonance magnétique nucléaire (RMN) du proton, du carbone 13 et par des expériences de corrélations homonucléaires et hétéronucléaires à deux dimensions.

Le composé le moins polaire (Rf = 0,7) est identifié à la (+) catéchine. Celui de polarité intermédiaire (Rf = 0,5) est identifié au procyanidol dimère B₃ (dimère de catéchine unis par les carbones en position 4 et 8). Le composé le plus polaire (Rf = 0,3) est identifié à un trimère de 3 unités catéchine liées par les atomes de carbone en position 4 et 8.

### C - Détermination de l'activité antiradicalaire

Les essais ont été effectués sur une culture de kératinocytes NCTC 2544 par une méthode permettant de mesurer la formation de malondialdéhyde (MDA) lors de l'exposition au t-butylhydroperoxyde comme oxydant. Le MDA est le principal produit de dégradation oxydative des lipides membranaires et autres composants cellulaires. La mesure du taux de réduction de la formation de MDA dans les cellules traitées par un produit est un bon indice de la capacité du produit à limiter la peroxydation lipidique.

A la dose de 0,002 % en poids par volume de culture de cellules, l'extrait de tormentille obtenu en A entraîne une réduction significative de la production de MDA de 18 %.

### D - Détermination de l'activité antiradicalaire d'un mélange de l'extrait de tormentille obtenu en A et d'un extrait de café vert.

On a mélangé en quantités égales l'extrait de tormentille obtenu en A et un extrait de café vert contenant 94,5 % en poids d'acides chlorogéniques, dont 50 % en poids d'acide 5-caféylquinique.

Un essai de mesure de l'activité antiradicalaire, tel que décrit en C, a montré que ce mélange provoque à la dose de 0,002 % poids/volume, une diminution de la production de MDA de 39 %. Des mesures effectuées avec l'extrait de café vert à la dose de 0,5 % poids/volume montrent que l'activité antiradicalaire de l'extrait de café vert seul est négligeable : la réduction de la production de MDA n'est que de 4 %.

## Revendications

1. Procédé d'extraction de polyphénols catéchiques essentiellement oligomères à partir de potentilles, caractérisé par le fait qu'il comporte les étapes suivantes :
a) la plante entière ou une partie de la plante est traitée par un solvant organique polaire, pur ou en mélange avec de l'eau,
b) l'extrait obtenu à l'étape a) est évaporé à sec à une température d'au plus 60°C,
c) le résidu d'évaporation de l'étape b) est repris par l'eau, puis la solution aqueuse obtenue est épuisée par un solvant non-miscible à l'eau et susceptible de dissoudre les polyphénols catéchiques oligomères,
d) la solution organique de l'étape c) est évaporée à sec à une température d'au plus 60°C.

2. Procédé selon la revendication 1, caractérisé par le fait que dans l'étape a), on utilise un solvant organique constitué par un alcool aliphatique en C₁-C₄.

3. Procédé selon l'une des revendications 1 ou 2, caractérisé par le fait que dans l'étape a), on mélange le solvant organique utilisé avec une quantité d'eau comprise entre 1 et 50 % en poids.

4. Procédé selon l'une des revendications 1 à 3, caractérisé par le fait que les étapes b) et/ou d) est (ou sont) effectuée(s) sous pression réduite.

5. Procédé selon l'une des revendications 1 à 4, caractérisé par le fait qu'à l'étape a), la durée de contact est comprise entre 10 mn et 2 heures.

6. Procédé selon l'une des revendications 1 à 5, caractérisé par le fait qu'à l'étape a), on broye la potentille ou partie de potentille avant traitement par le solvant.

7. Procédé selon l'une des revendications 1 à 6, caractérisé par le fait qu'à l'étape c), le solvant non-miscible à l'eau susceptible de dissoudre les polyphénols catéchiques oligomères est un acétate d'alkyl C₁-C₃.

8. Procédé selon la revendication 7, caractérisé par le fait que l'on utilise comme solvant à l'étape c) l'acétate d'éthyle, en un volume sensiblement équivalent à celui de la solution aqueuse et que l'on effectue plusieurs extractions successives.

9. Procédé selon l'une des revendications 1 à 8, caractérisé par le fait qu'à l'étape c), on sèche les phases solvant à l'aide d'un desséchant inerte.

10. Procédé selon l'une des revendications 1 à 9, caractérisé par le fait que l'on purifie l'extrait obtenu à l'étape d) par reprise par de l'eau distillée et qu'ensuite on le sèche.

11. Extrait contenant des polyphénols catéchiques essentiellement oligomères susceptible d'être obtenu par le procédé selon l'une des revendications 1 à 10.

12. Utilisation de l'extrait selon la revendication 11, comme composé actif dans la préparation de compositions pharmaceutiques, diététiques ou cosmétiques ayant des propriétés antiradicalaires et/ou de protection contre les rayons UV.

13. Utilisation selon la revendication 12, caractérisée par le fait que l'on mélange l'extrait selon la revendication il avec au moins un acide hydroxycinnamique.

## Claims

1. Process for extracting essentially oligomeric catechin polyphenols from potentillas, characterized in that it entails the following steps:
a) the whole plant or a part of the plant is treated with a polar organic solvent, pure or mixed with water,
b) the extract obtained in step a) is evaporated to dryness at a temperature of not more than 60°C,
c) the evaporation residue from step b) is taken up with water, and the aqueous solution obtained is then exhaustively extracted with a water-immiscible solvent capable of dissolving the oligomeric catechin polyphenols,
d) the organic solution from step c) is evaporated to dryness at a temperature of not more than 60°C.

2. Process according to Claim 1, characterized in that, in step a), an organic solvent consisting of a C1-C4 aliphatic alcohol is used.

3. Process according to either of Claims 1 and 2, characterized in that, in step a), the organic solvent used is mixed with an amount of water of between 1 and 50% by weight.

4. Process according to one of Claims 1 to 3, characterized in that steps b) and/or d) is/are carried out under reduced pressure.

5. Process according to one of Claims 1 to 4, characterized in that, in step a), the contact time is between 10 min and 2 hours.

6. Process according to one of Claims 1 to 5, characterized in that, in step a), the potentilla or potentilla part is ground before treatment with the solvent.

7. Process according to one of Claims 1 to 6, characterized in that, in step c), the water-immiscible solvent capable of dissolving the oligomeric catechin polyphenols is a C1-C3 alkyl acetate.

8. Process according to Claim 7, characterized in that ethyl acetate, in a volume substantially equivalent to that of the aqueous solution, is used as solvent in step c), and in that several successive extractions are carried out.

9. Process according to one of Claims 1 to 8, characterized in that, in step c), the solvent phases are dried using an inert drying agent.

10. Process according to one of Claims 1 to 9, characterized in that the extract obtained in step d) is purified by being taken up with distilled water, and in that it is then dried.

11. Extract containing essentially oligomeric catechin polyphenols, capable of being obtained by the process according to one of Claims 1 to 10.

12. Use of the extract according to Claim 11, as active compound in the preparation of pharmaceutical, dietetic or cosmetic compositions having free radical-scavenging properties and/or properties of protection against UV rays.

13. Use according to Claim 12, characterized in that the extract according to Claim 11 is mixed with at least one hydroxycinnamic acid.

## Patentansprüche

1. Verfahren zur Extraktion von katechinischen Polyphenolen, die im Wesentlichen oligomer sind, aus Fingerkraut, dadurch gekennzeichnet, dass es folgende Schritte umfasst:
a) die gesamte Pflanze oder ein Teil der Pflanze wird mit einem reinen oder mit Wasser gemischten polaren organischen Lösungsmittel behandelt,
b) der in Schritt a) erhaltene Extrakt wird bei einer Temperatur von über 60 °C zur Trockene eingedampft,
c) der Rückstand der Verdampfung aus Schritt b) wird in Wasser aufgenommen, anschließend wird die erhaltene wässrige Lösung mit einem in Wasser nicht mischbaren und zum Lösen der oligomeren katechinischen Polyphenole geeigneten Lösungsmittel extrahiert,
d) die organische Lösung aus Schritt c) wird bei einer Temperatur von über 60 °C zur Trockene eingedampft.

2. Verfahren nach Anspruch 1, dadurch gekennzeichnet, dass man in Schritt a) als organisches Lösungsmittel einen aliphatischen (C₁-C₄) -Alkohol verwendet.

3. Verfahren nach einem der Ansprüche 1 oder 2, dadurch gekennzeichnet, dass man in Schritt a) das verwendete organische Lösungsmittel mit Wasser in einer Menge von 1 bis 50 Gew.-% mischt.

4. Verfahren nach einem der Ansprüche 1 bis 3, dadurch gekennzeichnet, dass man die Schritte b) und/oder d) unter vermindertem Druck durchführt.

5. Verfahren nach einem der Ansprüche 1 bis 4, dadurch gekennzeichnet, dass in Schritt a) die Zeitdauer des Kontaktes zwischen 10 min und 2 h beträgt.

6. Verfahren nach einem der Ansprüche 1 bis 5, dadurch gekennzeichnet, dass in Schritt a) das Fingerkraut oder ein Teil des Fingerkrautes vor der Behandlung mit dem Lösungsmittel zerkleinert wird.

7. Verfahren nach einem der Ansprüche 1 bis 6, dadurch gekennzeichnet, dass in Schritt c) das in Wasser nicht mischbare, zum Lösen der oligomeren katechinischen Polyphenole geeignete Lösungsmittel ein (C₁-C₃)-Alkylacetat ist.

8. Verfahren nach Anspruch 7, dadurch gekennzeichnet, dass man in Schritt c) als Lösungsmittel ein der wässrigen Phase entsprechendes Volumen an Ethylacetat verwendet und dass man mehrere aufeinander folgende Extraktionen durchführt.

9. Verfahren nach einem der Ansprüche 1 bis 8, dadurch gekennzeichnet, dass in Schritt c) die organischen Phasen mit einem inerten Trockenmittel getrocknet werden.

10. Verfahren nach einem der Ansprüche 1 bis 9, dadurch gekennzeichnet, dass der in Schritt d) erhaltene Extrakt durch Wiederaufnahme in destilliertem Wasser gereinigt und anschließend getrocknet wird.

11. Extrakt, der katechinische Polyphenole enthält, die im Wesentlichen oligomer sind, und der durch ein Verfahren nach einem der Ansprüche 1 bis 10 erhältlich ist.

12. Verwendung des Extrakts nach Anspruch 11 als aktive Verbindung bei der Herstellung von pharmazeutischen, diätetischen oder kosmetischen Zusammensetzungen mit Eigenschaften eines Radikalfängers und/oder UV-Schut zmittels.

13. Verwendung nach Anspruch 12, dadurch gekennzeichnet, dass der Extrakt gemäß Anspruch 11 mit mindestens einer Hydroxyzimtsäure vermengt wird.
